# EUROPEAN PATENT APPLICATION

(11) **EP 3 882 831 A1**
(43) Date of publication of application: **22.09.2021**
(21) Application number: 20164439.0
(22) Date of filing: 20.03.2020
(51) Int. Cl.: G06Q 10/06, G06Q 50/26

(54) **SMART QUARANTINE SYSTEM AND SMART QUARANTINE METHOD**

(30) Priority: 19.03.2020 KR 20200033617
(71) Applicant: Ministry for Health & Welfare, Sejong-si 30113 (KR); Infomining Co., Ltd., Gyeonggi-do 14548 (KR)
(72) Inventor: GO, Deuk Young, 30113 Sejong-si (KR); SIN, Ji Myeong, 30113 Sejong-si (KR); KIM, Jong Deok, 30113 Sejong-si (KR); NA, Kyung Chae, 30113 Sejong-si (KR); YOON, Jong Hyun, 30113 Sejong-si (KR); PARK, Jung Ha, 30113 Sejong-si (KR); KIM, Hyuk Soo, 30113 Sejong-si (KR); LEE, Jae Yong, 14585 Bucheon-si, Gyeonggi-do (KR)
(74) Representative: M. Zardi & Co S.A.

(57) **Abstract**

A smart quarantine system includes an artificial intelligence control system which receives information about an emigration and immigration person, stores at least a portion of the information about the emigration and immigration person, compare the at least a portion of the information about the emigration and immigration person with pre-stored information, and create a complete barcode based on the comparison result, a symptom identification unit to identify a normal or abnormal health state of the emigration and immigration person, based on the complete barcode, and a quarantine execution unit to execute customized quarantine based on the normal or abnormal health state of the emigration and immigration person. The quarantine execution unit includes: an isolation and examination recommendation unit to recommend isolation and examination such that the emigration and immigration person is isolated and examined, when a health state of the emigration and immigration person is abnormal, an immigration approval unit to approve immigration of the emigration and immigration person when the health state of the emigration and immigration person is normal, and a self-diagnosis system to perform self-diagnosis of a health of the emigration and immigration person after the immigration.

## Description

### BACKGROUND

Embodiments of the inventive concept described herein relate a smart quarantine system and a smart quarantine method, and, more specifically, to a smart quarantine system and a smart quarantine method capable of quickly and accurately and efficiently quarantining an emigration and immigration person.

Emigration and immigration refers to exiting and entering a certain country. In a global age, it is difficult to quarantine quickly and accurately emigration and immigration persons because thousands of emigration and immigration peoples together exit and enter a specific country. The quarantine of the emigration and immigration person refers to identification of presence or absence of a pathogen in a person entering or leaving a country to prevent quarantine infectious diseases.

A certain disease has an incubation period. Thus, it is necessary to manage persons having the disease for a certain period of time. However, it is economically and temporally difficult to manage a large number of people having the disease over a period of time. Further, even when it is possible to manage a large number of people having the disease, but when voluntary participation of emigration and immigration people is absent, there is a problem that it is difficult to efficiently manage the large number of people having the disease over a period of time.

Referring to current management status of an infectious disease, there is a problem that a system for management, monitoring, and information-sharing of an infected person is insufficient. Further, infections may spread further due to safety-insensitivity to infectious diseases. For example, at timing of emigration and immigration, initial recognition of the infected person is not possible, and thus the infected person may spread. Thus, necessity of artificial intelligence based quarantine of the emigration and immigration person is emerging.

### [Prior Art Documents]

### [Patent Documents]

Japanese Patent Application Publication No. 2011-65540 (2011.03.31)

### SUMMARY

Embodiments of the inventive concept provide a smart quarantine system capable of performing quarantine of an emigration and immigration person quickly and accurately and efficiently.

Embodiments of the inventive concept provide a smart quarantine method capable of performing quarantine of an emigration and immigration person quickly and accurately and efficiently.

The purposes to be achieved by the inventive concept are not limited to the purposes as mentioned above, and other purposes as not mentioned will be clearly understood by a person skilled in the art from following descriptions.

According to an exemplary embodiment, a smart quarantine system includes an artificial intelligence control system which receives information about an emigration and immigration person, stores at least a portion of the information about the emigration and immigration person, compares the at least a portion of the information about the emigration and immigration person with pre-stored information, and creates a complete barcode based on the comparison result, a symptom identification unit that identifies a normal or abnormal health state of the emigration and immigration person, based on the complete barcode, and a quarantine execution unit that executes customized quarantine based on the normal or abnormal health state of the emigration and immigration person. The quarantine execution unit includes: an isolation and examination recommendation unit to recommend isolation and examination such that the emigration and immigration person is isolated and examined, when a health state of the emigration and immigration person is abnormal, an immigration approval unit to approve immigration of the emigration and immigration person when the health state of the emigration and immigration person is normal, and a self-diagnosis system to perform self-diagnosis of a health of the emigration and immigration person after the immigration.

According to an exemplary embodiment, a smart quarantine method includes receiving information about an emigration and immigration person, storing at least a portion of the information about the emigration and immigration person, comparing the at least a portion of the information about the emigration and immigration person with pre-stored information, creating a complete barcode based on the comparison result, identifying a normal or abnormal health state of the emigration and immigration person, based on the complete barcode, and executing customized quarantine based on the normal or abnormal health state of the emigration and immigration person. The executing of the customized quarantine includes recommending isolation and examination such that the emigration and immigration person is isolated and examined, when a health state of the emigration and immigration person is abnormal, approving immigration of the emigration and immigration person when a health state of the emigration and immigration person is normal, and operating a self-diagnosis system such that the person performs self-diagnosis of a health of the emigration and immigration person after the immigration.

### BRIEF DESCRIPTION OF DRAWINGS

The above and other objects and features will become apparent from the following description with reference to the following figures, wherein like reference numerals refer to like parts throughout the various figures unless otherwise specified, and wherein:
FIG. 1 is a block diagram schematically showing a smart quarantine system according to an embodiment of the inventive concept;
FIG. 2 is a table illustratively showing a function and a function-implementation scheme that may be performed by an artificial intelligence control system included in a smart quarantine system according to an embodiment of the inventive concept.
FIG. 3a is a table showing, by way of example, information about an emigration and immigration person identified by an artificial intelligence control system included in a smart quarantine system according to an embodiment of the inventive concept when the emigration and immigration person has a smart device.
FIG. 3b is a table showing, by way of example, information about an emigration and immigration person identified by an artificial intelligence control system included in a smart quarantine system according to an embodiment of the inventive concept when the emigration and immigration person does not carry a smart device.
FIG. 3c is a table showing, by way of example, information about an emigration and immigration person identified by an artificial intelligence control system included in a smart quarantine system according to an embodiment of the inventive concept when the emigration and immigration person does not carry a smart device or the artificial intelligence control system may not contact a domestic acquaintance using an artificial intelligence phone function.
FIG. 4 is a schematic flow diagram of a self-diagnosis method performed by a self-diagnosis system included in a smart quarantine system according to an embodiment of the inventive concept.

### DETAILED DESCRIPTION

Advantages and features of the inventive concept, and methods of achieving them will become apparent with reference to embodiments described below in detail in conjunction with the accompanying drawings. However, the inventive concept is not limited to the embodiments disclosed below, but may be implemented in various forms. The present embodiments are provided to merely complete the disclosure of the inventive concept, and to merely fully inform those skilled in the art of the inventive concept of the scope of the inventive concept. The inventive concept is only defined by the scope of the claims.

The terminology used herein is for the purpose of describing the embodiments only and is not intended to limit the inventive concept. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof. The same reference numbers in different figures denote the same or similar elements, and as such perform similar functionality. As used herein, the term "and/or" includes any and all combinations of one or more of the associated listed items. Although "first", "second", etc. are used to describe various components, these components are not limited by these terms. These terms are only used to distinguish one component from another. Therefore, a first component mentioned below may be a second component within a technical spirit of the inventive concept.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, embodiments of the inventive concept will be described in detail with reference to the accompanying drawings.

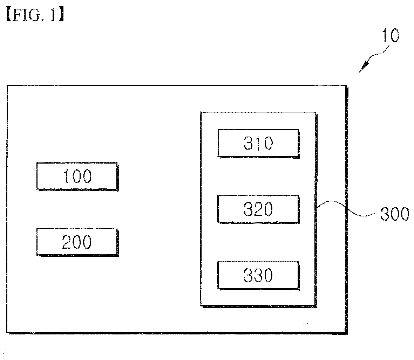

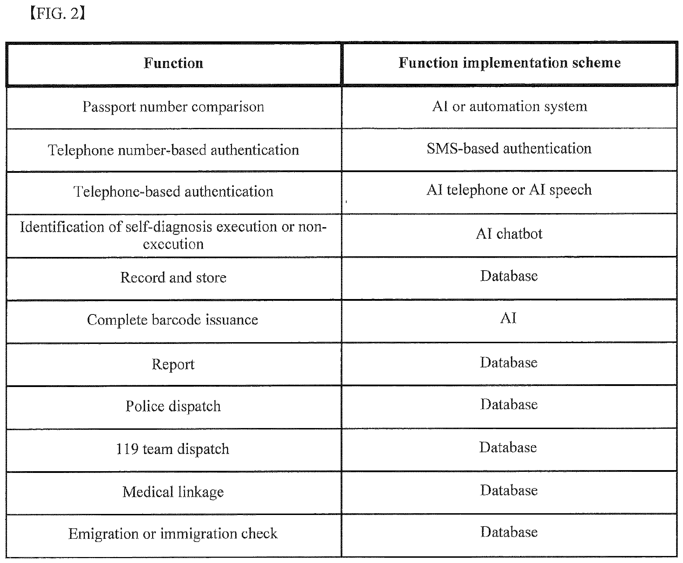

FIG. 1 is a block diagram schematically showing a smart quarantine system according to an embodiment of the inventive concept. FIG. 2 is an exemplary table showing a function and a function implementation scheme that may be performed by an artificial intelligence control system included in a smart quarantine system according to an embodiment of the inventive concept.

Referring to FIG. 1 and FIG. 2, a smart quarantine system 10 according to an embodiment of the inventive concept includes an artificial intelligence control system 100, a symptom identification unit 200, and a quarantine execution unit 300.

The artificial intelligence control system 100, the symptom identification unit 200, and the quarantine execution unit 300 may be connected to each other in a wired or wireless manner. For example, the artificial intelligence control system 100, the symptom identification unit 200, and the quarantine execution unit 300 may be connected to each other via IoT (Internet of Things), Wi-Fi, NFC (Near-field communication), RTLS (Real-time locating system), community mapping, Web 2.0 platform, etc.

The artificial intelligence control system 100 receives information about an emigration and immigration person. The information about the emigration and immigration person may be input, for example, through an application installed on a smart device of the emigration and immigration person. For example, the information of the input emigration and immigration person may be input to the artificial intelligence control system 100 via Internet of Things, Wi-Fi, NFC (Near-field communication), RTLS (Real-time locating system), community mapping, Web 2.0 platform, etc..

The artificial intelligence control system 100 stores therein at least a portion of information about the emigration and immigration person. The artificial intelligence control system 100 compares the at least a portion of the information about the emigration and immigration person with pre-stored information. The pre-stored information as used herein may be stored in database included in the artificial intelligence control system 100 in one example. The pre-stored information may be, for example, information provided to the artificial intelligence control system 100 from an airline corporation or a Ministry of Justice department of a government.

The artificial intelligence control system 100 stores the at least a portion of the information about the emigration and immigration person, and compares the at least a portion of the information about the emigration and immigration person with the pre-stored information to create a complete barcode.

The artificial intelligence control system 100 may perform, for example, passport number matching, phone number authentication, phone authentication, self-diagnosis identification, recording and storage, complete barcode issuance, reporting, police dispatch, 119 dispatch, medical linkage, emigration and immigration identification functions, etc. For example, the artificial intelligence control system 100 may compare a passport number of the emigration and immigration person with a pre-stored passport number in an artificial intelligence or automation manner. For example, the artificial intelligence control system 100 may authenticate a phone number of the emigration and immigration person via SMS authentication. For example, the artificial intelligence control system 100 may identify an acquaintance (family, friend, or the like) of the emigration and immigration person using an artificial intelligence phone or artificial intelligence speech. In the artificial intelligence phone and the artificial intelligence speech, a real person does not perform a phone call or speech, but an artificial intelligence machine or an artificial intelligence program performs a phone call or speech.

For example, the artificial intelligence control system 100 may use an artificial intelligence chatbot to identify whether self-diagnosis is performed by the emigration and immigration person. The artificial intelligence chatbot may mean that the chatting is performed by an artificial intelligence machine or an artificial intelligence program rather than a real person.

For example, the artificial intelligence control system 100 may store contents recorded by the emigration and immigration person in database. The contents recorded by the emigration and immigration person may include an immigration purpose.

For example, when the emigration and immigration person is a criminal or performs abnormal behavior, the artificial intelligence control system 100 may report a police of the emigration and immigration person for police dispatch.

For example, when an abnormality of the health of the emigration and immigration person is determined, the artificial intelligence control system 100 reports this situation using a phone number 119 for an emergency team dispatch or reports the same to a medical institution for medical linkage.

For example, the artificial intelligence control system 100 may identify past emigration and immigration history of the emigration and immigration person using the database.

For example, the artificial intelligence control system 100 may issue the complete barcode in an artificial intelligence manner. For example, the complete barcode may contain entire information about the immigration record of the emigration and immigration person. The entire immigration record information may include past immigration records of the emigration and immigration person.

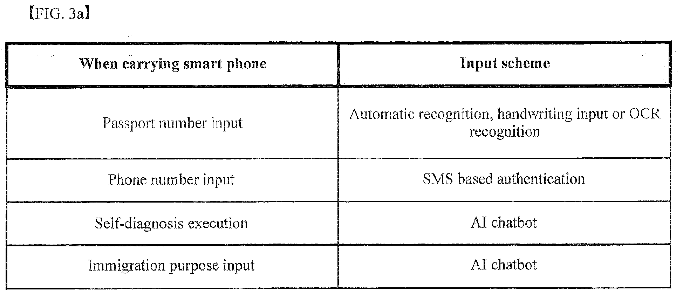

FIG. 3a is a table showing, by way of example, information about an emigration and immigration person identified by an artificial intelligence control system included in a smart quarantine system according to an embodiment of the inventive concept when the emigration and immigration person has a smart device.

Referring to FIG. 1, FIG. 2, and FIG. 3a, when the emigration and immigration person has a smart device, information about the emigration and immigration person as received by the artificial intelligence control system 100 may include a passport number, a mobile phone number, self-diagnosis execution or non-execution, and an immigration purpose of the emigration and immigration person.

For example, the artificial intelligence control system 100 may receive the passport number of the emigration and immigration person in at least one of an automatic recognition manner, a handwriting input manner, and an optical character recognition (OCR) manner, and may compare the received passport number with a pre-stored passport number.

For example, the artificial intelligence control system 100 may compare the mobile phone number with a pre-stored mobile phone number in a text authentication manner.

For example, the artificial intelligence control system 100 may receive whether the self-diagnosis is executed and the immigration purpose using an artificial intelligence chatbot and may store the same. For example, the artificial intelligence control system 100 may store whether the self-diagnosis is executed and the immigration purpose in database.

For example, the artificial intelligence control system 100 may compare the passport number with the pre-stored passport number, and compare the mobile phone number with the pre-stored mobile phone number in a text authentication manner, and store whether the self-diagnosis is executed and the immigration purpose. Then, the artificial intelligence control system 100 may issue the complete barcode in an artificial intelligence manner.

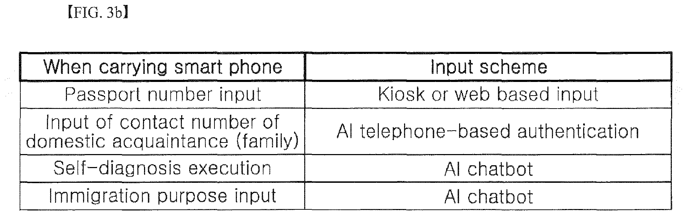

FIG. 3b is a table showing, by way of example, information about an emigration and immigration person identified by an artificial intelligence control system included in a smart quarantine system according to an embodiment of the inventive concept when the emigration and immigration person does not carry a smart device.

Referring to FIG. 1, FIG. 2, and FIG. 3b, when the emigration and immigration person does not have a smart device, the information about the emigration and immigration person received by the artificial intelligence control system 100 may include the passport number, contact information of a domestic acquaintance, whether the self-diagnosis is executed, and the immigration purpose.

For example, the artificial intelligence control system 100 may receive a passport number using a kiosk or in a web-based input manner, and compare the received passport number with the pre-stored passport number.

For example, the artificial intelligence control system 100 may contact the domestic acquaintance contact using an artificial intelligence phone function to identify the acquaintance.

For example, the artificial intelligence control system 100 may receive whether the self-diagnosis is executed and the immigration purpose using an artificial intelligence chatbot and may store the same. For example, the artificial intelligence control system 100 may store whether the self-diagnosis is executed and the immigration purpose in database.

For example, the artificial intelligence control system 100 may compare the passport number with the pre-stored passport number, and may contact the contact information of the domestic acquaintance using the artificial intelligence phone function to identify the acquaintance, and then may store whether the self-diagnosis is executed and the immigration purpose. Then, the artificial intelligence control system 100 may issue the complete barcode in an artificial intelligence manner.

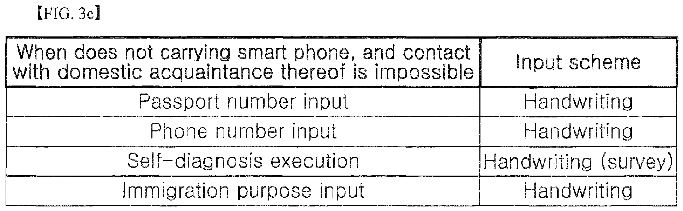

FIG. 3c is a table showing, by way of example, information about an emigration and immigration person identified by an artificial intelligence control system included in a smart quarantine system according to an embodiment of the inventive concept when the emigration and immigration person does not carry a smart device or the artificial intelligence control system may not contact a domestic acquaintance using an artificial intelligence phone function.

Referring to FIG. 1, FIG. 2, and FIG. 3c, when the emigration and immigration person does not have a smart device or the artificial intelligence control system 100 may not contact a domestic acquaintance using an artificial intelligence phone function, the information about the emigration and immigration person received by the artificial intelligence control system 100 may include the passport number, a domestic residence contact, whether the self-diagnosis is executed, and the immigration purpose.

For example, the artificial intelligence control system 100 may receive the passport number, the domestic residence contact, whether the self-diagnosis is executed, and the immigration purpose as inputted by the emigration and immigration person in a handwriting manner, and then may compare the same with a pre-stored handwriting version thereof acquired from the emigration and immigration person in a letter recognition artificial intelligence manner.

For example, the artificial intelligence control system 100 may receive the passport number, the domestic residence contact, whether the self-diagnosis is executed, and the immigration purpose as inputted by the emigration and immigration person in a handwriting manner, and then may compare the same with a pre-stored handwriting version thereof acquired from the emigration and immigration person in a letter recognition artificial intelligence manner, and then may issue the complete barcode in an artificial intelligence manner.

Referring back to FIG. 1, the symptom identification unit 200 identifies a normal or abnormal health state of the emigration and immigration person based on the complete barcode. For example, the complete barcode has different electronic marks based on whether the health of the emigration and immigration person is normal or abnormal. Thus, the symptom identification unit 200 may identify the marks.

The quarantine execution unit 300 provides customized quarantine based on the normal or abnormal health state of the emigration and immigration person. The quarantine execution unit 300 may include an isolation and examination recommendation unit 310, an immigration approval unit 320, and a self-diagnosis system 330.

When the health state of the emigration and immigration person is abnormal, the isolation and examination recommendation unit 310 may recommend isolation and examination so that the emigration and immigration person may be isolated and examined. For example, the isolation and examination recommendation unit 310 may provide an isolation and examination recommendation alarm, barcode, message, or telephone for a smart device of the emigration and immigration person or an emigration and immigration management office in an artificial intelligence manner.

When the health state of the emigration and immigration person is normal, the immigration approval unit 320 may approval immigration of the emigration and immigration person. For example, when the health state of the emigration and immigration person is normal, the immigration approval unit 320 may provide an immigration-approval alarm, barcode, message, or telephone for the smart device of the emigration and immigration person or the emigration and immigration management office in an artificial intelligence manner.

The self-diagnosis system 330 may allow the emigration and immigration person to perform self-diagnosis of the health of the emigration and immigration person after immigration. The self-diagnosis system 330 will be described later in more detail.

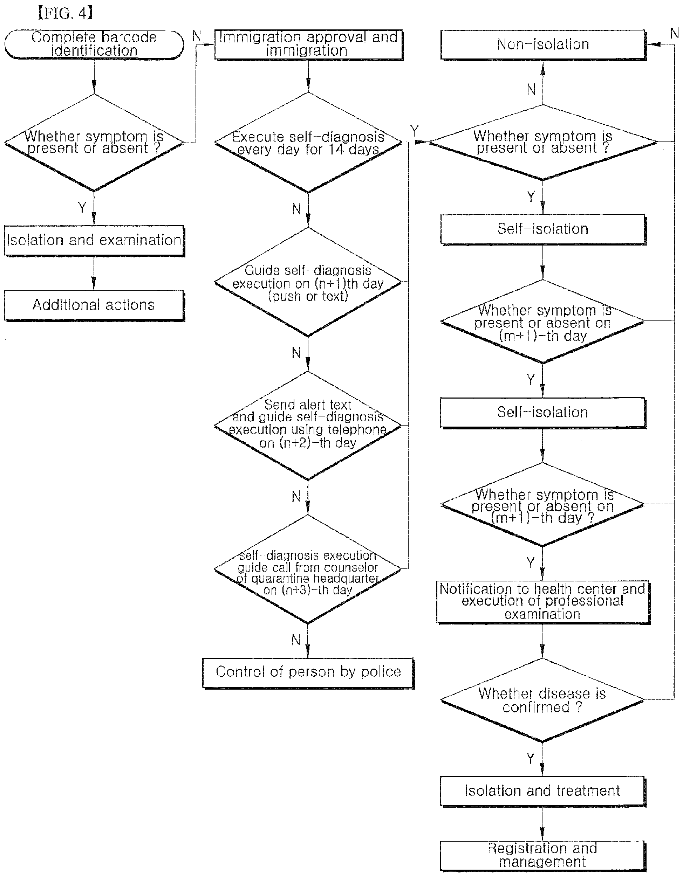

FIG. 4 is a schematic flow diagram of a self-diagnosis method performed by a self-diagnosis system included in a smart quarantine system according to an embodiment of the inventive concept.

Referring to FIG. 1 to FIG. 4, the self-diagnosis system 330 provides the emigration and immigration person with a self-diagnosis application such that the emigration and immigration person performs self-diagnosis thereof for 14 days since an immigration day or a next day thereto. The term " self-diagnosis" means that the emigration and immigration person directly checks a symptom such as a body temperature, cough, difficulty breathing, misdiagnosis, sore throat, runny nose, muscle pain, vomiting, diarrhea, etc. using a diagnosis device such as a thermometer, an application installed on the smart device, etc.

A wearable device as the diagnosis device may be provided to the emigration and immigration person belonging to a risk group. The term "risk group" may mean a group of persons whose symptom of an infectious disease such as a body temperature, cough, difficulty breathing, misdiagnosis, sore throat, runny nose, muscle pain, vomiting, and diarrhea has a level higher than a predefined level. The term "wearable device" refers to a device that may be worn by the emigration and immigration person, and may include a smart watch or the like.

The wearable device may measure, for example, pulse data and body temperature data of the emigration and immigration person, and may provide the data for the self-diagnosis system 330. For example, the wearable device may be connected to the self-diagnosis system 330 via Internet of Things, Wi-Fi, NFC (Near-field communication), RTLS (Real-time locating system), community mapping, Web 2.0 platform, etc.

The emigration and immigration person not belonging to the risk group may measure, for example, the pulse data and the body temperature data thereof, using a smart device possessed by the emigration and immigration person and may provide the data for the self-diagnosis system 330. For example, an application may be installed on the smart device possessed by the emigration and immigration person who in turn may measure the pulse data and the body temperature data of the emigration and immigration person using the installed application. For example, the smart device may be connected to the self-diagnosis system 330 via Internet of Things, Wi-Fi, NFC (Near-field communication), RTLS (Real-time locating system), community mapping, Web 2.0 platform, etc.

The self-diagnosis system 330 may receive the self-diagnosis data about the emigration and immigration person. The self-diagnosis system 330 may receive the self-diagnosis data about the emigration and immigration person via direct input by the emigration and immigration person, via input and transmission using the smart device of the emigration and immigration person, or via transmission using the wearable device of the emigration and immigration person.

When the self-diagnosis system 330 identifies that abnormality in the health of the emigration and immigration person has not been detected for 14 days from the immigration day or the next day thereto, the self-diagnosis system 330 may approve non-isolation. For example, the self-diagnosis system 330 may provide an alarm, barcode, message, chatbot, or phone that approves non-isolation for the smart device of the emigration and immigration person in an artificial intelligence manner.

For example, the self-diagnosis system 330 may receive the self-diagnosis data about the emigration and immigration person every day for 14 days from the immigration day or the next day thereto. However, the present disclosure is not limited thereto. The self-diagnosis system 330 may receive the self-diagnosis data of the emigration and immigration person every two days or every three days for 14 days from the immigration day or the next day thereto.

For example, when the self-diagnosis system 330 does not receive the self-diagnosis data about the emigration and immigration person on a specified date within 14 days from the immigration day or the next day thereto, the self-diagnosis system 330 may guide the emigration and immigration person to perform the self-diagnosis on a next day ((n+1)-th day) to a last day (n-th day) of a duration for which the self-diagnosis data is not received by the self-diagnosis system 330. In this connection, the duration is n days. For example, the self-diagnosis system 330 may send a self-diagnosis execution guide text or notification to the emigration and immigration person.

For example, when the self-diagnosis system 330 does not receive the self-diagnosis data about the emigration and immigration person although the self-diagnosis execution guide text or notification is sent to the emigration and immigration person, the self-diagnosis system 330 may send an alert text and the self-diagnosis execution guide to the emigration and immigration person using an artificial intelligence phone function on a (n+2)-th day

For example, when the self-diagnosis system 330 does not receive the self-diagnosis data about the emigration and immigration person although the self-diagnosis system 330 sends the alert text and the self-diagnosis execution guide to the emigration and immigration person, the self-diagnosis system 330 may provide telephone-based guideline information so that a counselor provides a telephone-based guideline on a (n+3)-th day. The telephone-based guideline information may mean a telephone request notification, barcode, or message so that the counselor provides the telephone-based guideline.

For example, when the self-diagnosis system 330 does not receive the self-diagnosis data about the emigration and immigration person although the emigration and immigration person has received the telephone-based guideline from the counselor, the self-diagnosis system 330 may report this situation of a police. For example, the self-diagnosis system 330 may provide the police with an alarm, barcode, messages, chatbot, or telephone to control the emigration and immigration person in an artificial intelligence manner.

For example, when an abnormality occurs in the health of the emigration and immigration person within 14 days from the immigration day or the next day thereto, the self-diagnosis system 330 may recommend self-isolation. For example, when the abnormity occurs in the health of the emigration and immigration person, the self-diagnosis system 330 may provide an alarm, barcode, message, chatbot, or phone that recommends the self-isolation for the smart device of the emigration and immigration person in an artificial intelligence manner. When the emigration and immigration person violates the self-isolation recommendation, the self-diagnosis system 330 may provide an alarm, barcode, message, chatbot, or telephone for recommending self-isolation enforcement for a government agency, a police station, and the like in an artificial intelligence manner.

For example, the self-diagnosis system 330 may receive the self-diagnosis data about the emigration and immigration person on a (m+1)-th day as a next day to a last day of a duration for which the emigration and immigration person has been isolated. In this connection, the duration for which the emigration and immigration person has been isolated due to the abnormal health state is m days. When the self-diagnosis system 330 receives the self-diagnosis data about the emigration and immigration person, and determines that the health state of the emigration and immigration person is normal, the self-diagnosis system 330 may approve non-isolation. For example, when the self-diagnosis system 330 determines that the health state of the emigration and immigration person is normal, the self-diagnosis system 330 may provide an alarms, barcode, message, chatbot, or phone that approves the non-isolation for the smart device of the emigration and immigration person in an artificial intelligence manner.

For example, when the self-diagnosis system 330 determines that the health state of the emigration and immigration person is abnormal, the self-diagnosis system 330 may maintain self-isolation. For example, when the self-diagnosis system 330 determines that the health state of the emigration and immigration person is abnormal, the self-diagnosis system 330 may provide an alarm, barcode, message, chatbot, or phone that recommends maintaining the self-isolation for the smart device of the emigration and immigration person in an artificial intelligence manner.

For example, the self-diagnosis system 330 may receive the self-diagnosis data about the emigration and immigration person on a (m+2)-th day. In this connection, the duration for which the emigration and immigration person has been isolated due to the abnormal health state is m days. When the self-diagnosis system 330 receives the self-diagnosis data about the emigration and immigration person, and the health state of the emigration and immigration person is normal, non-isolation may be approved by the self-diagnosis system 330. For example, when the self-diagnosis system 330 determines that the health state of the emigration and immigration person is normal, the self-diagnosis system 330 may provide an alarm, barcode, message, chatbot, or phone that approves the non-isolation to the smart device of the emigration and immigration person in an artificial intelligence manner.

For example, when the self-diagnosis system 330 determines that the health state of the emigration and immigration person is abnormal, the self-diagnosis system 330 may inform a public community health center of this situation so that the emigration and immigration person is subjected to professional examination. For example, when the self-diagnosis system 330 determines that the health state of the emigration and immigration person is abnormal, the self-diagnosis system 330 may provide an alarm, barcode, message, chatbot, or telephone for recommending the professional examination for the public community health center such that the professional examination of the emigration and immigration person is carried out by the public community health center.

For example, the self-diagnosis system 330 may receive examination data about the health state of the emigration and immigration person from the public community health center. For example, when the self-diagnosis system 330 receives the examination data from the public community health center, and the health state of the emigration and immigration person is normal, non-isolation may be approved by the self-diagnosis system 330. For example, when the self-diagnosis system 330 determines that the health state of the emigration and immigration person is normal, the self-diagnosis system 330 may provide an alarm, barcode, message, chatbot, or phone that approves the non-isolation to the smart device of the emigration and immigration person or the public community health center in an artificial intelligence manner.

For example, when the self-diagnosis system 330 determines that the health state of the emigration and immigration person is abnormal, the self-diagnosis system 330 may allow the public community health center to isolate and control the emigration and immigration person. For example, when the self-diagnosis system 330 determines that the health state of the emigration and immigration person is abnormal, the self-diagnosis system 330 may provide an alarm, barcode, message, chatbot, or telephone that recommends the isolation and treatment of the emigration and immigration person for the smart device of the emigration and immigration person or the public community health center in an artificial intelligence manner.

When the emigration and immigration person belongs to a risk group, the self-diagnosis system 330 may receive the self-diagnosis data and the health data collected using the wearable device. For example, the self-diagnosis system 330 receives the self-diagnosis data and the health data collected using the wearable device on the (m+2)-th day. In this connection, the duration for which the emigration and immigration person has been isolated due to the abnormal health state is m days. Then, when the health state of the emigration and immigration person is normal, non-isolation may be approved by the self-diagnosis system 330. For example, when the self-diagnosis system 330 determines that the health state of the emigration and immigration person is normal, the self-diagnosis system 330 may provide an alarm, barcode, message, chatbot, or phone that approves the non-isolation for the smart device of the emigration and immigration person in an artificial intelligence manner.

For example, when the self-diagnosis system 330 determines that the health state of the emigration and immigration person is abnormal, the self-diagnosis system 330 may inform the public community health center of this situation so that the emigration and immigration person is subjected to the professional examination. For example, when the self-diagnosis system 330 determines that the health state of the emigration and immigration person is abnormal, the self-diagnosis system 330 may provide an alarm, barcode, message, chatbot, or telephone for recommending the professional examination for the public community health center such that the professional examination of the emigration and immigration person is carried out by the public community health center.

The smart quarantine system 10 according to an embodiment of the inventive concept may be implemented in the artificial intelligence manner. Even when an infectious disease has an incubation period, and voluntary participation of the emigration and immigration person is absent, the smart quarantine system 10 according to an embodiment of the inventive concept may perform quarantine of the emigration and immigration person quickly and accurately and efficiently.

Hereinafter, a smart quarantine method according to an embodiment of the inventive concept will be described. Hereinafter, differences between configurations of the smart quarantine system and the smart quarantine method according to an embodiment of the inventive concept will be mainly described. Thus, details as not described below may be the same as those of the smart quarantine system according to an embodiment of the inventive concept.

Referring to FIG. 1 to FIG. 4, the smart quarantine method according to an embodiment of the inventive concept includes creating the complete barcode; identifying a normal or abnormal health state; and performing quarantine.

Creating the complete barcode includes receiving information about the emigration and immigration person. Creating the complete barcode may be performed by the artificial intelligence control system 100. The information about the emigration and immigration person may be input, for example, via an application installed on the smart device of the emigration and immigration person. For example, the information of the input emigration and immigration person may be provided to the artificial intelligence control system 100 via Internet of Things, Wi-Fi, NFC (Near-field communication), RTLS (Real-time locating system), community mapping, Web 2.0 platform, etc..

Creating the complete barcode includes storing at least a portion of the information about the emigration and immigration person. Creating the complete barcode includes comparing the at least a portion of the information about the emigration and immigration person with the pre-stored information. The pre-stored information herein may be stored in database included in the artificial intelligence control system 100 in one example. The pre-stored information may be, for example, information provided to the artificial intelligence control system 100 from an airline corporation or the Ministry of Justice.

In the step of creating the complete barcode, the at least a portion of the information about the emigration and immigration person is stored, and then the at least a portion of the information about the emigration and immigration person is compared with the pre-stored information to create the complete barcode.

In the step of creating the complete barcode, the artificial intelligence control system 100 may perform, for example, passport number matching, phone number authentication, phone authentication, self-diagnosis identification, recording and storage, complete barcode issuance, report, police dispatch, 119 team dispatch, medical linkage, emigration and immigration identification, etc. For example, the artificial intelligence control system 100 may compare the passport number of the emigration and immigration person with the pre-stored passport number in an artificial intelligence or automation manner. For example, the artificial intelligence control system 100 may authenticate the phone number of the emigration and immigration person in a SMS authentication manner. For example, the artificial intelligence control system 100 may identify a contact number of an acquaintance (family, friend) of the emigration and immigration person using an artificial intelligence phone or an artificial intelligence speech. In the artificial intelligence phoning and the artificial intelligence speech, a real person does not perform a phone call or speech, but an artificial intelligence machine or an artificial intelligence program performs a phone call or speech.

For example, in the step of creating the complete barcode, the artificial intelligence control system 100 may use an artificial intelligence chatbot to identify whether self-diagnosis is performed by the emigration and immigration person. The artificial intelligence chatbot may mean that the chatting is performed by an artificial intelligence machine or an artificial intelligence program rather than a real person.

For example, in the step of creating the complete barcode, the artificial intelligence control system 100 may store contents recorded by the emigration and immigration person in database. The contents recorded by the emigration and immigration person may include an immigration purpose.

For example, in the step of creating the complete barcode, when the emigration and immigration person is a criminal or performs abnormal behavior, the artificial intelligence control system 100 may report a police of the emigration and immigration person for police dispatch.

For example, in the step of creating the complete barcode, when an abnormality of the health of the emigration and immigration person is determined, the artificial intelligence control system 100 reports this situation using a phone number 119 for an emergency team dispatch or reports the same to a medical institution for medical linkage.

For example, in the step of creating the complete barcode, the artificial intelligence control system 100 may identify past emigration and immigration history of the emigration and immigration person using the database.

For example, in the step of creating the complete barcode, the artificial intelligence control system 100 may issue the complete barcode in an artificial intelligence manner. For example, the complete barcode may contain entire information about the immigration record of the emigration and immigration person. The entire immigration record information may include past immigration records of the emigration and immigration person.

Referring to FIG. 1, FIG. 2, and FIG. 3a, in the step of creating the complete barcode, when the emigration and immigration person has a smart device, information about the emigration and immigration person as received by the artificial intelligence control system 100 may include a passport number, a mobile phone number, self-diagnosis execution or non-execution, and an immigration purpose of the emigration and immigration person.

For example, in the step of creating the complete barcode, the artificial intelligence control system 100 may receive the passport number of the emigration and immigration person in at least one of an automatic recognition manner, a handwriting input manner, and an optical character recognition (OCR) manner, and may compare the received passport number with a pre-stored passport number.

For example, in the step of creating the complete barcode, the artificial intelligence control system 100 may compare the mobile phone number with a pre-stored mobile phone number in a text authentication manner.

For example, in the step of creating the complete barcode, the artificial intelligence control system 100 may receive whether the self-diagnosis is executed and the immigration purpose using an artificial intelligence chatbot and may store the same. For example, in the step of creating the complete barcode, the artificial intelligence control system 100 may store whether the self-diagnosis is executed and the immigration purpose in database.

For example, in the step of creating the complete barcode, the artificial intelligence control system 100 may compare the passport number with the pre-stored passport number, and compare the mobile phone number with the pre-stored mobile phone number in a text authentication manner, and store whether the self-diagnosis is executed and the immigration purpose. Then, the artificial intelligence control system 100 may issue the complete barcode in an artificial intelligence manner.

Referring to FIG. 1, FIG. 2, and FIG. 3b, in the step of creating the complete barcode, when the emigration and immigration person does not have a smart device, the information about the emigration and immigration person received by the artificial intelligence control system 100 may include the passport number, contact information of a domestic acquaintance, whether the self-diagnosis is executed, and the immigration purpose.

For example, in the step of creating the complete barcode, the artificial intelligence control system 100 may receive a passport number using a kiosk or in a web-based input manner, and compare the received passport number with the pre-stored passport number.

For example, in the step of creating the complete barcode, the artificial intelligence control system 100 may contact the domestic acquaintance contact using an artificial intelligence phone function to identify the acquaintance.

For example, in the step of creating the complete barcode, the artificial intelligence control system 100 may receive whether the self-diagnosis is executed and the immigration purpose using an artificial intelligence chatbot and may store the same. For example, the artificial intelligence control system 100 may store whether the self-diagnosis is executed and the immigration purpose in database.

For example, in the step of creating the complete barcode, the artificial intelligence control system 100 may compare the passport number with the pre-stored passport number, and may contact the contact information of the domestic acquaintance using the artificial intelligence phone function to identify the acquaintance, and then may store whether the self-diagnosis is executed and the immigration purpose. Then, the artificial intelligence control system 100 may issue the complete barcode in an artificial intelligence manner.

Referring to FIG. 1, FIG. 2, and FIG. 3c, in the step of creating the complete barcode, when the emigration and immigration person does not have a smart device, the information about the emigration and immigration person received by the artificial intelligence control system 100 may include the passport number, a domestic residence contact, whether the self-diagnosis is executed, and the immigration purpose.

For example, in the step of creating the complete barcode, the artificial intelligence control system 100 may receive the passport number, the domestic residence contact, whether the self-diagnosis is executed, and the immigration purpose as inputted by the emigration and immigration person in a handwriting manner, and then may compare the same with a pre-stored handwriting version thereof acquired from the emigration and immigration person in a letter recognition artificial intelligence manner.

For example, in the step of creating the complete barcode, the artificial intelligence control system 100 may receive the passport number, the domestic residence contact, whether the self-diagnosis is executed, and the immigration purpose as inputted by the emigration and immigration person in a handwriting manner, and then may compare the same with a pre-stored handwriting version thereof acquired from the emigration and immigration person in a letter recognition artificial intelligence manner, and then may issue the complete barcode in an artificial intelligence manner.

Referring back to FIG. 1 to FIG. 4, in the step of identifying the normal or abnormal state of the emigration and immigration person, the symptom identification unit 200 identifies the normal or abnormal health state of the emigration and immigration person based on the complete barcode. For example, the complete barcode has different electronic marks based on whether the health of the emigration and immigration person is normal or abnormal. Thus, the symptom identification unit 200 may identify the marks.

In the step of performing the quarantine, the quarantine execution unit 300 provides customized quarantine based on the normal or abnormal health state of the emigration and immigration person. The step of performing the quarantine may be executed by the quarantine execution unit 300 including the isolation and examination recommendation unit 310, the immigration approval unit 320, and the self-diagnosis system 330.

The step of performing the quarantine may include an isolation and examination recommendation step, an immigration approval step, and a step of operating the self-diagnosis system. In the isolation and examination recommendation step, when the health state of the emigration and immigration person is abnormal, the isolation and examination recommendation unit 310 may recommend isolation and examination so that the emigration and immigration person may be isolated and examined. For example, the isolation and examination recommendation unit 310 may provide an isolation and examination recommendation alarm, barcode, message, or telephone for a smart device of the emigration and immigration person or an emigration and immigration management office in an artificial intelligence manner.

In the immigration approval step, when the health state of the emigration and immigration person is normal, the immigration approval unit 320 may approval immigration of the emigration and immigration person. For example, when the health state of the emigration and immigration person is normal, the immigration approval unit 320 may provide an immigration-approval alarm, barcode, message, or telephone for the smart device of the emigration and immigration person or the emigration and immigration management office in an artificial intelligence manner.

In the step of operating the self-diagnosis system, the emigration and immigration person may perform self-diagnosis of the health of the emigration and immigration person after immigration. The step of operating the self-diagnosis system may be based on the self-diagnosis system 330 as described above. The step of operating the self-diagnosis system will be described later in more detail.

In the step of operating the self-diagnosis system, the self-diagnosis system 330 provides the emigration and immigration person with a self-diagnosis application such that the emigration and immigration person performs self-diagnosis thereof for 14 days since an immigration day or a next day thereto. In the step of operating the self-diagnosis system, the wearable device as the diagnosis device may be provided to the emigration and immigration person belonging to the risk group.

In the step of operating the self-diagnosis system, the emigration and immigration person not belonging to the risk group may measure, for example, the pulse data and the body temperature data thereof, using a smart device possessed by the emigration and immigration person and may provide the data for the self-diagnosis system 330. For example, in the step of operating the self-diagnosis system, an application may be installed on the smart device possessed by the emigration and immigration person who in turn may measure the pulse data and the body temperature data of the emigration and immigration person using the installed application.

In the step of operating the self-diagnosis system, the self-diagnosis system 330 may receive the self-diagnosis data about the emigration and immigration person. In the step of operating the self-diagnosis system, the self-diagnosis system 330 may receive the self-diagnosis data about the emigration and immigration person via direct input by the emigration and immigration person, via input and transmission using the smart device of the emigration and immigration person, or via transmission using the wearable device of the emigration and immigration person.

In the step of operating the self-diagnosis system, when the self-diagnosis system 330 identifies that abnormality in the health of the emigration and immigration person has not been detected for 14 days from the immigration day or the next day thereto, the self-diagnosis system 330 may approve non-isolation. For example, In the step of operating the self-diagnosis system, the self-diagnosis system 330 may provide an alarm, barcode, message, chatbot, or phone that approves non-isolation for the smart device of the emigration and immigration person in an artificial intelligence manner.

For example, In the step of operating the self-diagnosis system, the self-diagnosis system 330 may receive the self-diagnosis data about the emigration and immigration person every day for 14 days from the immigration day or the next day thereto. However, the present disclosure is not limited thereto. In the step of operating the self-diagnosis system, the self-diagnosis system 330 may receive the self-diagnosis data of the emigration and immigration person every two days or every three days for 14 days from the immigration day or the next day thereto.

For example, in the step of operating the self-diagnosis system, when the self-diagnosis system 330 does not receive the self-diagnosis data about the emigration and immigration person on a specified date within 14 days from the immigration day or the next day thereto, the self-diagnosis system 330 may guide the emigration and immigration person to perform the self-diagnosis on a next day ((n+l)-th day) to a last day (n-th day) of a duration for which the self-diagnosis data is not received by the self-diagnosis system 330. In this connection, the duration is n days. For example, in the step of operating the self-diagnosis system, the self-diagnosis system 330 may send a self-diagnosis execution guide text or notification to the emigration and immigration person.

For example, in the step of operating the self-diagnosis system, when the self-diagnosis system 330 does not receive the self-diagnosis data about the emigration and immigration person although the self-diagnosis execution guide text or notification is sent to the emigration and immigration person, the self-diagnosis system 330 may send an alert text and the self-diagnosis execution guide to the emigration and immigration person using an artificial intelligence phone function.

For example, in the step of operating the self-diagnosis system, when the self-diagnosis system 330 does not receive the self-diagnosis data about the emigration and immigration person although the self-diagnosis system 330 sends the alert text and the self-diagnosis execution guide to the emigration and immigration person, the self-diagnosis system 330 may provide telephone-based guideline information so that a counselor provides a telephone-based guideline on a (n+3)-th day. The telephone-based guideline information may mean a telephone request notification, barcode, or message so that the counselor provides the telephone-based guideline.

For example, in the step of operating the self-diagnosis system, when the self-diagnosis system 330 does not receive the self-diagnosis data about the emigration and immigration person although the emigration and immigration person has received the telephone-based guideline from the counselor, the self-diagnosis system 330 may report this situation of a police. For example, the self-diagnosis system 330 may provide the police with an alarm, barcode, messages, chatbot, or telephone to control the emigration and immigration person in an artificial intelligence manner.

For example, in the step of operating the self-diagnosis system, when an abnormality occurs in the health of the emigration and immigration person within 14 days from the immigration day or the next day thereto, the self-diagnosis system 330 may recommend self-isolation. For example, in the step of operating the self-diagnosis system, when the abnormity occurs in the health of the emigration and immigration person, the self-diagnosis system 330 may provide an alarm, barcode, message, chatbot, or phone that recommends the self-isolation for the smart device of the emigration and immigration person in an artificial intelligence manner. In the step of operating the self-diagnosis system, when the emigration and immigration person violates the self-isolation recommendation, the self-diagnosis system 330 may provide an alarm, barcode, message, chatbot, or telephone for recommending self-isolation enforcement for a government agency, a police station, and the like in an artificial intelligence manner.

For example, in the step of operating the self-diagnosis system, the self-diagnosis system 330 may receive the self-diagnosis data about the emigration and immigration person on a (m+1)-th day as a next day to a last day of a duration for which the emigration and immigration person has been isolated. In this connection, the duration for which the emigration and immigration person has been isolated due to the abnormal health state is m days. In the step of operating the self-diagnosis system, when the self-diagnosis system 330 receives the self-diagnosis data about the emigration and immigration person, and determines that the health state of the emigration and immigration person is normal, the self-diagnosis system 330 may approve non-isolation. For example, in the step of operating the self-diagnosis system, when the self-diagnosis system 330 determines that the health state of the emigration and immigration person is normal, the self-diagnosis system 330 may provide an alarms, barcode, message, chatbot, or phone that approves the non-isolation for the smart device of the emigration and immigration person in an artificial intelligence manner.

For example, in the step of operating the self-diagnosis system, when the self-diagnosis system 330 determines that the health state of the emigration and immigration person is abnormal, the self-diagnosis system 330 may maintain self-isolation. For example, in the step of operating the self-diagnosis system, when the self-diagnosis system 330 determines that the health state of the emigration and immigration person is abnormal, the self-diagnosis system 330 may provide an alarm, barcode, message, chatbot, or phone that recommends maintaining the self-isolation for the smart device of the emigration and immigration person in an artificial intelligence manner.

For example, in the step of operating the self-diagnosis system, the self-diagnosis system 330 may receive the self-diagnosis data about the emigration and immigration person on a (m+2)-th day. In this connection, the duration for which the emigration and immigration person has been isolated due to the abnormal health state is m days. In the step of operating the self-diagnosis system, when the self-diagnosis system 330 receives the self-diagnosis data about the emigration and immigration person, and the health state of the emigration and immigration person is normal, non-isolation may be approved by the self-diagnosis system 330. For example, in the step of operating the self-diagnosis system, when the self-diagnosis system 330 determines that the health state of the emigration and immigration person is normal, the self-diagnosis system 330 may provide an alarm, barcode, message, chatbot, or phone that approves the non-isolation to the smart device of the emigration and immigration person in an artificial intelligence manner.

For example, in the step of operating the self-diagnosis system, when the self-diagnosis system 330 determines that the health state of the emigration and immigration person is abnormal, the self-diagnosis system 330 may inform a public community health center of this situation so that the emigration and immigration person is subjected to professional examination. For example, in the step of operating the self-diagnosis system, when the self-diagnosis system 330 determines that the health state of the emigration and immigration person is abnormal, the self-diagnosis system 330 may provide an alarm, barcode, message, chatbot, or telephone for recommending the professional examination for the public community health center such that the professional examination of the emigration and immigration person is carried out by the public community health center.

For example, in the step of operating the self-diagnosis system, the self-diagnosis system 330 may receive examination data about the health state of the emigration and immigration person from the public community health center. For example, in the step of operating the self-diagnosis system, when the self-diagnosis system 330 receives the examination data from the public community health center, and the health state of the emigration and immigration person is normal, non-isolation may be approved by the self-diagnosis system 330. For example, in the step of operating the self-diagnosis system, when the self-diagnosis system 330 determines that the health state of the emigration and immigration person is normal, the self-diagnosis system 330 may provide an alarm, barcode, message, chatbot, or phone that approves the non-isolation to the smart device of the emigration and immigration person or the public community health center in an artificial intelligence manner.

For example, in the step of operating the self-diagnosis system, when the self-diagnosis system 330 determines that the health state of the emigration and immigration person is abnormal, the self-diagnosis system 330 may allow the public community health center to isolate and treat the emigration and immigration person. For example, in the step of operating the self-diagnosis system, when the self-diagnosis system 330 determines that the health state of the emigration and immigration person is abnormal, the self-diagnosis system 330 may provide an alarm, barcode, message, chatbot, or telephone that recommends the isolation and treatment of the emigration and immigration person for the smart device of the emigration and immigration person or the public community health center in an artificial intelligence manner.

In the step of operating the self-diagnosis system, when the emigration and immigration person belongs to a risk group, the self-diagnosis system 330 may receive the self-diagnosis data and the health data collected using the wearable device. For example, in the step of operating the self-diagnosis system, the self-diagnosis system 330 receives the self-diagnosis data and the health data collected using the wearable device on the (m+2)-th day. In this connection, the duration for which the emigration and immigration person has been isolated due to the abnormal health state is m days. Then, when the health state of the emigration and immigration person is normal, non-isolation may be approved by the self-diagnosis system 330. For example, in the step of operating the self-diagnosis system, when the self-diagnosis system 330 determines that the health state of the emigration and immigration person is normal, the self-diagnosis system 330 may provide an alarm, barcode, message, chatbot, or phone that approves the non-isolation for the smart device of the emigration and immigration person in an artificial intelligence manner.

For example, in the step of operating the self-diagnosis system, when the self-diagnosis system 330 determines that the health state of the emigration and immigration person is abnormal, the self-diagnosis system 330 may inform the public community health center of this situation so that the emigration and immigration person is subjected to the professional examination. For example, in the step of operating the self-diagnosis system, when the self-diagnosis system 330 determines that the health state of the emigration and immigration person is abnormal, the self-diagnosis system 330 may provide an alarm, barcode, message, chatbot, or telephone for recommending the professional examination for the public community health center such that the professional examination of the emigration and immigration person is carried out by the public community health center.

The smart quarantine method according to an embodiment of the inventive concept may be performed in the artificial intelligence manner. Even when an infectious disease has an incubation period, and voluntary participation of the emigration and immigration person is absent, the smart quarantine method according to an embodiment of the inventive concept may perform quarantine of the emigration and immigration person quickly and accurately and efficiently.

According to the inventive concept, the smart quarantine system that may quickly and accurately and efficiently perform quarantine of an emigration and immigration person may be realized.

According to the inventive concept, the smart quarantine method that may quickly and accurately and efficiently perform quarantine of an emigration and immigration person may be realized.

While the inventive concept has been described with reference to exemplary embodiments, it will be apparent to those skilled in the art that various changes and modifications may be made without departing from the spirit and scope of the inventive concept. Therefore, it should be understood that the above embodiments are not limiting, but illustrative.

### REFERENCE NUMERALS

- 10:: Smart quarantine system
- 100:: Artificial intelligence control system
- 200:: Symptom identification unit
- 300:: Quarantine execution unit
- 310:: Isolation and examination recommendation unit
- 320:: Immigration approval unit
- 330:: Self-diagnosis system

## Claims

1. A smart quarantine system comprising:
an artificial intelligence control system configured to:
receive information about an emigration and immigration person;
store at least a portion of the information about the emigration and immigration person;
compare the at least a portion of the information about the emigration and immigration person with pre-stored information; and
create a complete barcode based on the comparison result,
a symptom identification unit configured to identify a normal or abnormal health state of the emigration and immigration person, based on the complete barcode; and
a quarantine execution unit configured to execute customized quarantine based on the normal or abnormal health state of the emigration and immigration person,
wherein the quarantine execution unit includes:
an isolation and examination recommendation unit configured to recommend isolation and examination such that the emigration and immigration person is isolated and examined, when a health state of the emigration and immigration person is abnormal;
an immigration approval unit configured to approve immigration of the emigration and immigration person when the health state of the emigration and immigration person is normal; and
a self-diagnosis system configured to perform self-diagnosis of a health of the emigration and immigration person after the immigration.

2. The smart quarantine system of claim 1, wherein the information about the emigration and immigration person includes:
a passport number,
self-diagnosis execution or non-execution; and
an immigration purpose.

3. The smart quarantine system of claim 2, wherein when the emigration and immigration person carries a smart device, the information about the emigration and immigration person further includes a phone number related to the smart device,
wherein the artificial intelligence control system is configured to:
receive the passport number in at least one of an automatic recognition manner, a handwriting input manner by the emigration and immigration person, or an optical character recognition (OCR) manner;
compare the received passport number with a pre-stored passport number;
compare the phone number with a pre-stored phone number in a text authentication manner; and
receive whether the self-diagnosis is executed and the immigration purpose using an artificial intelligence chatbot, and store the received self-diagnosis execution or non-execution and immigration purpose.

4. The smart quarantine system of claim 2, wherein when the emigration and immigration person does not carry a smart device, the information about the emigration and immigration person further includes a contact of a domestic acquaintance of the emigration and immigration person,
wherein the artificial intelligence control system is configured to:
receive the passport number using a kiosk or in a web-based input manner;
compare the received passport number with a pre-stored passport number;
contact the domestic acquaintance based the contact using an artificial intelligence phone function to identify the acquaintance; and
receive whether the self-diagnosis is executed and the immigration purpose using an artificial intelligence chatbot.

5. The smart quarantine system of claim 2, wherein when the emigration and immigration person does not carry a smart device, or the artificial intelligence control system does not contact a domestic acquaintance of the emigration and immigration person using an artificial intelligence phone function,
the information about the emigration and immigration person further includes a domestic residence contact of the emigration and immigration person,
wherein the artificial intelligence control system is configured to:
receive the passport number, the domestic residence contact, whether the self-diagnosis is executed, and the immigration purpose as inputted by the emigration and immigration person in a handwriting manner; and
compare the received passport number, domestic residence contact, self-diagnosis execution or non-execution, and immigration purpose with a pre-stored handwriting version thereof acquired from the emigration and immigration person in a letter recognition artificial intelligence manner.

6. The smart quarantine system of claim 1, wherein the self-diagnosis system is configured to:
provide the emigration and immigration person with a self-diagnosis application such that the emigration and immigration person performs self-diagnosis thereof for 14 days from an immigration day or a next day thereto;
receive self-diagnosis data about the emigration and immigration person; and
upon determination based on the data that an abnormal health sate of the emigration and immigration person has not been detected for 14 days from the immigration day or the next day thereto, approve non-isolation.

7. The smart quarantine system of claim 6, wherein the self-diagnosis system is configured to:
when the self-diagnosis system does not receive the self-diagnosis data about the emigration and immigration person on within 14 days from the immigration day or the next day thereto, guide the emigration and immigration person to perform self-diagnosis on a next day ((n+1)-th day) to a last day (n-th day) of a duration for which the self-diagnosis data has not been received by the self-diagnosis system, wherein the duration is n days.

8. The smart quarantine system of claim 7, wherein the self-diagnosis system is configured to:
when the self-diagnosis system does not receive the self-diagnosis data about the emigration and immigration person although the self-diagnosis execution guide is sent to the emigration and immigration person, send an alert text and the self-diagnosis execution guide to the emigration and immigration person using an artificial intelligence phone function on a (n+2)-th day.

9. The smart quarantine system of claim 8, wherein the self-diagnosis system is configured to:
when the self-diagnosis system does not receive the self-diagnosis data about the emigration and immigration person although the self-diagnosis system sends the alert text and the self-diagnosis execution guide to the emigration and immigration person, provide telephone-based guideline information so that a counselor provides a telephone-based guideline on a (n+3)-th day.

10. The smart quarantine system of claim 9, wherein the self-diagnosis system is configured to:
when the self-diagnosis system does not receive the self-diagnosis data about the emigration and immigration person although the emigration and immigration person has received the telephone-based guideline from the counselor, send information to a police to control the person.

11. The smart quarantine system of claim 6, wherein the self-diagnosis system is configured to:
when an abnormality occurs in the health of the emigration and immigration person within 14 days from the immigration day or the next day thereto, recommend self-isolation of the person.

12. The smart quarantine system of claim 11, wherein the self-diagnosis system is configured to:
when the health state of the emigration and immigration person is normal on a (m+1)-th day as a next day to a last day of a duration for which the emigration and immigration person has been isolated, approve non-isolation of the person, wherein the duration is m days; or
when the health state of the emigration and immigration person is abnormal on the (m+l)-th day, maintain the self-isolation of the person.

13. The smart quarantine system of claim 12, wherein the self-diagnosis system is configured to:
when the health state of the emigration and immigration person is normal on a (m+2)-th day, approve non-isolation of the person; or
when the health state of the emigration and immigration person is abnormal on the (m+2)-th day, inform a public community health center of the abnormal state so that the emigration and immigration person is subjected to professional examination.

14. The smart quarantine system of claim 13, wherein the self-diagnosis system is configured to:
receive examination data about the health state of the emigration and immigration person from the public community health center;
upon determination, based on the examination data, that the health state of the emigration and immigration person is normal, approve non-isolation of the person, or
upon determination, based on the examination data, that the health state of the emigration and immigration person is abnormal, allow the public community health center to isolate and treat the person.

15. The smart quarantine system of claim 14, wherein the emigration and immigration person belongs to a risk group,
wherein the self-diagnosis system is configured to:
when the health state of the emigration and immigration person is normal on a (m+2)-th day, approve non-isolation of the person; or
when the health state of the emigration and immigration person is abnormal on the (m+2)-th day, inform a public community health center of the abnormal state so that the emigration and immigration person is subjected to professional examination; and
send pulse data and body temperature data of the emigration and immigration person to the public community health center, wherein the pulse data and the body temperature data is received from a wearable device provided to the emigration and immigration person.

## Amended claims

### Amended claims in accordance with Rule 137(2) EPC.

1. A smart quarantine system (10) comprising:
an artificial intelligence control system (100) configured to:
receive information about an emigration and immigration person;
store at least a portion of the information about the emigration and immigration person;
compare the at least a portion of the information about the emigration and immigration person with pre-stored information; and
create a complete barcode based on the comparison result,
a symptom identification unit (200) configured to identify a normal or abnormal health state of the emigration and immigration person, based on the complete barcode; and
a quarantine execution unit (300) configured to execute customized quarantine based on the normal or abnormal health state of the emigration and immigration person,
**characterized in that** the quarantine execution unit (300) includes:
an isolation and examination recommendation unit (310) configured to recommend isolation and examination such that the emigration and immigration person is isolated and examined, when a health state of the emigration and immigration person is abnormal;
an immigration approval unit (320) configured to approve immigration of the emigration and immigration person when the health state of the emigration and immigration person is normal; and
a self-diagnosis system (330) configured to perform self-diagnosis of a health of the emigration and immigration person after the immigration,
wherein the information about the emigration and immigration person includes:
a passport number,
self-diagnosis execution or non-execution; and
an immigration purpose, and
wherein when the emigration and immigration person carries a smart device, the information about the emigration and immigration person further includes a phone number related to the smart device,
wherein the artificial intelligence control system (100) is configured to:
receive the passport number in at least one of an automatic recognition manner, a handwriting input manner by the emigration and immigration person, or an optical character recognition (OCR) manner;
compare the received passport number with a pre-stored passport number;
compare the phone number with a pre-stored phone number in a text authentication manner; and
receive whether the self-diagnosis is executed and the immigration purpose using an artificial intelligence chatbot, and store the received self-diagnosis execution or non-execution and immigration purpose.

2. The smart quarantine system (10) of claim 1, wherein when the emigration and immigration person does not carry a smart device, the information about the emigration and immigration person further includes a contact of a domestic acquaintance of the emigration and immigration person,
wherein the artificial intelligence control system (100) is configured to:
receive the passport number using a kiosk or in a web-based input manner;
compare the received passport number with a pre-stored passport number;
contact the domestic acquaintance based the contact using an artificial intelligence phone function to identify the acquaintance; and
receive whether the self-diagnosis is executed and the immigration purpose using an artificial intelligence chatbot.

3. The smart quarantine system (10) of claim 1, wherein when the emigration and immigration person does not carry a smart device, or the artificial intelligence control system (100) does not contact a domestic acquaintance of the emigration and immigration person using an artificial intelligence phone function,
the information about the emigration and immigration person further includes a domestic residence contact of the emigration and immigration person,
wherein the artificial intelligence control system (100) is configured to:
receive the passport number, the domestic residence contact, whether the self-diagnosis is executed, and the immigration purpose as inputted by the emigration and immigration person in a handwriting manner; and
compare the received passport number, domestic residence contact, self-diagnosis execution or non-execution, and immigration purpose with a pre-stored handwriting version thereof acquired from the emigration and immigration person in a letter recognition artificial intelligence manner.

4. The smart quarantine system (10) of claim 1, wherein the self-diagnosis system is configured to:
provide the emigration and immigration person with a self-diagnosis application such that the emigration and immigration person performs self-diagnosis thereof for 14 days from an immigration day or a next day thereto;
receive self-diagnosis data about the emigration and immigration person; and
upon determination based on the data that an abnormal health sate of the emigration and immigration person has not been detected for 14 days from the immigration day or the next day thereto, approve non-isolation.

5. The smart quarantine system (10) of claim 4, wherein the self-diagnosis system is configured to:
when the self-diagnosis system does not receive the self-diagnosis data about the emigration and immigration person on within 14 days from the immigration day or the next day thereto, guide the emigration and immigration person to perform self-diagnosis on a next day ((n+1)-th day) to a last day (n-th day) of a duration for which the self-diagnosis data has not been received by the self-diagnosis system, wherein the duration is n days.

6. The smart quarantine system (10) of claim 5, wherein the self-diagnosis system is configured to:
when the self-diagnosis system does not receive the self-diagnosis data about the emigration and immigration person although the self-diagnosis execution guide is sent to the emigration and immigration person, send an alert text and the self-diagnosis execution guide to the emigration and immigration person using an artificial intelligence phone function on a (n+2)-th day.

7. The smart quarantine system (10) of claim 6, wherein the self-diagnosis system is configured to:
when the self-diagnosis system does not receive the self-diagnosis data about the emigration and immigration person although the self-diagnosis system sends the alert text and the self-diagnosis execution guide to the emigration and immigration person, provide telephone-based guideline information so that a counselor provides a telephone-based guideline on a (n+3)-th day.

8. The smart quarantine system (10) of claim 7, wherein the self-diagnosis system is configured to:
when the self-diagnosis system does not receive the self-diagnosis data about the emigration and immigration person although the emigration and immigration person has received the telephone-based guideline from the counselor, send information to a police to control the person.

9. The smart quarantine system (10) of claim 4, wherein the self-diagnosis system is configured to:
when an abnormality occurs in the health of the emigration and immigration person within 14 days from the immigration day or the next day thereto, recommend self-isolation of the person.

10. The smart quarantine system (10) of claim 9, wherein the self-diagnosis system is configured to:
when the health state of the emigration and immigration person is normal on a (m+1)-th day as a next day to a last day of a duration for which the emigration and immigration person has been isolated, approve non-isolation of the person, wherein the duration is m days; or
when the health state of the emigration and immigration person is abnormal on the (m+1)-th day, maintain the self-isolation of the person.

11. The smart quarantine system (10) of claim 10, wherein the self-diagnosis system is configured to:
when the health state of the emigration and immigration person is normal on a (m+2)-th day, approve non-isolation of the person; or
when the health state of the emigration and immigration person is abnormal on the (m+2)-th day, inform a public community health center of the abnormal state so that the emigration and immigration person is subjected to professional examination.

12. The smart quarantine system (10) of claim 11, wherein the self-diagnosis system is configured to:
receive examination data about the health state of the emigration and immigration person from the public community health center;
upon determination, based on the examination data, that the health state of the emigration and immigration person is normal, approve non-isolation of the person, or
upon determination, based on the examination data, that the health state of the emigration and immigration person is abnormal, allow the public community health center to isolate and treat the person.

13. The smart quarantine system (10) of claim 12, wherein the emigration and immigration person belongs to a risk group,
wherein the self-diagnosis system is configured to:
when the health state of the emigration and immigration person is normal on a (m+2)-th day, approve non-isolation of the person; or
when the health state of the emigration and immigration person is abnormal on the (m+2)-th day, inform a public community health center of the abnormal state so that the emigration and immigration person is subjected to professional examination; and
send pulse data and body temperature data of the emigration and immigration person to the public community health center, wherein the pulse data and the body temperature data is received from a wearable device provided to the emigration and immigration person.
